# FASCICULE DE BREVET EUROPEEN

(11) **EP 0 913 147 B1**
(45) Date de publication et mention de la délivrance du brevet: **31.05.2000**
(21) Numéro de dépôt: 98401659.2
(22) Date de dépôt: 02.07.1998
(51) Int. Cl.: A61K 7/48, A61K 7/06

(54) **Système à base de dérivé d'acide phosphonique et de métabisulfite pour stabiliser l'acide ascorbique**
System bestehend aus Phosphonsäurederivate und Metabisulfit zur Stabilisierung von Ascorbinsäure
System comprising a phosphonic acid derivative and a metabisulfit for stabilization of ascorbic acid

(30) Priorité: 02.09.1997 FR 9710903
(43) Date de publication de la demande: 06.05.1999
(73) Titulaire: L'OREAL, 75008 Paris (FR)
(72) Inventeur: Nguyen, Quang Lan, 92160 Antony (FR); Afriat, Isabelle, 75014 Paris (FR); Pham, Dang Man, 92800 Puteaux (FR); Chanvin, Florence, 91450 Soisy/S/Seine (FR)
(74) Mandataire: Tezier Herman, Béatrice

(56) Documents cités:
- EP-A- 0 670 157
- DATABASE WPI Week 9440 Derwent Publications Ltd., London, GB; AN 94-322095 XP002067095 & JP 06 247855 A (WAKO PURE CHEM IND)
- DATABASE WPI Week 8914 Derwent Publications Ltd., London, GB; AN 89-102027 XP002067096 & HU 46 540 A (HOFFMANN L.)

## Description

L'invention se rapporte à un système à base d'un dérivé d'acide phosphonique et d'un métabisulfite, pour stabiliser l'acide ascorbique, à l'utilisation de ce système dans des compositions contenant de l'acide ascorbique, et aux compositions le contenant en particulier dans les domaines cosmétique et/ou dermatologique.

L'invention se rapporte aussi à l'utilisation des compositions obtenues pour le traitement cosmétique de la peau humaine ainsi que pour la préparation d'une crème ou pommade destinée au traitement dermatologique de la peau humaine. Ces composition peuvent être appliquées par voie topique, sur le visage, y compris autour des yeux, sur le corps ainsi que sur le cuir chevelu des êtres humains.

On cherche depuis longtemps à stabiliser l'acide ascorbique, ou vitamine C, dans des présentations galéniques appropriées, du fait de ses propriétés bénéfiques.

En effet, l'acide ascorbique possède de nombreuses fonctions biologiques comme la stimulation de la synthèse du collagène, le renfort des tissus cutanés contre les agressions extérieures (rayonnements UV, pollution), la dépigmentation, l'activité anti-radicaux libres, la compensation de la déficience en vitamine E. Certaines de ces propriétés bénéfiques ont été rapportées notamment par ENGLAND ET SEIFTER dans l'article "The biochemical functions of ascorbic acid" paru dans Ann. Rev. Nutri.,1986, vol. 6, p. 365-406.

Cependant, en raison de sa structure chimique (d'alpha-cétolactone) l'acide ascorbique est très sensible à l'influence des paramètres de l'environnement comme la lumière, l'oxygène, l'eau (par son pH et par la présence de trace de métaux). Il s'ensuit une dégradation inéluctable au cours du temps de l'acide ascorbique en solution. Cette dégradation se traduit par une modification de la couleur et par la génération de dioxyde de carbone.

Dans l'état de la technique ce problème a été diversement traité.

Pour diminuer ou retarder la dégradation de l'acide ascorbique en solution, il a été préconisé dans le document US-A-5140043 de le stabiliser en l'introduisant dans des solutions hydroalcooliques, formées d'au moins 80 % d'eau et ayant un pH inférieur à 3,5. En raison de la forte acidité de ces solutions, leur utilisation dans le domaine cosmétique et/ou pharmaceutique est difficilement envisageable. En effet, une application répétée de ces solutions peut perturber l'équilibre de la peau et en particulier irriter, voire brûler la peau. En outre, à pH acide, si l'évolution de la couleur est stabilisée, la génération de dioxyde de carbone (C02) ne l'est pas.

On connaît, par ailleurs, l'article de B.R. Hajratwala intitulé "Stability of ascorbic acid", paru dans la Revue Sciences Pharmaceutiques, le 15 Mars 1985. Dans cet article, il est enseigné notamment de stabiliser l'acide ascorbique en solution aqueuse acide par ajout d'un agent tensioactif qui est un ester de sorbitanne oxyéthyléné. En particulier, l'auteur y a rapporté qu'à pH 3,4 et à 25 °C, l'ajout de cet agent diminuait la vitesse d'oxydation, donc de dégradation de l'acide ascorbique en solution. En outre, ce document enseigne l'emploi d'agent chélatant tel que l'éthylène diamine tétraacétique (EDTA), et le conditionnement sous azote en l'absence de lumière, pour améliorer la stabilité de l'acide ascorbique en solution aqueuse. Une telle solution aqueuse acide, appliquée sur la peau, présente les mêmes inconvénients que ceux décrits ci-dessus pour des solutions hydroalcooliques acides. De plus, la stabilisation obtenue est encore insuffisante.

D'autres modes de stabilisation de l'acide ascorbique ont été envisagés notamment par enrobage (technique décrite dans le document FR-A-1600826) ou par granulation de l'acide ascorbique (technique illustrée dans le document JP-A-53-127819, pour l'agro-alimentaire). Mais ces techniques sont, d'une part, coûteuses et peuvent, d'autre part, altérer l'acide ascorbique, par exemple lors d'un chauffage, et/ou conduire à des compositions peu cosmétiques, comme c'est le cas des granulés.

On connaît, par ailleurs, du document FR-A-1489249 l'emploi de sels métalliques d'acide ascorbique phosphorylé, notamment l'ascorbylphosphate de magnésium, dans des compositions cosmétiques. Ce dernier composé a une activité proche de celle de l'acide ascorbique dont il est issu mais il présente certains inconvénients qui rendent son utilisation sur la peau peu probable. En particulier, l'ascorbylphosphate de magnésium n'étant stable qu'en pH basique (pH 8 à pH 9), il doit être incorporé dans une composition basique qui peut être irritante pour la peau (dont le pH a une valeur d'environ 5,5).

En outre, le document EP-A-670157, décrit une émulsion contenant de l'acide ascorbique stabilisée. Toutefois, il s'agit d'une forme galénique particulière (émulsion eau-dans-huile) et il est souhaitable de pouvoir stabiliser l'acide ascorbique dans des compositions se présentant sous des formes galéniques très diverses.

En conséquence, l'ensemble des propositions qui ont été faites jusqu'ici n'a pas permis de résoudre les problèmes techniques liés à l'instabilité de l'acide ascorbique, dans toute forme galénique pour les domaines cosmétique et/ou dermatologique et à un coût compatible avec les exigences industrielles.

Il subsiste donc le besoin d'une composition utilisable notamment dans les domaines cosmétique et/ou dermatologique, contenant de l'acide ascorbique stabilisé, à l'état libre, c'est à dire sans groupement additionnel notamment stabilisant, qui ne provoque aucune irritation de la peau après application, qui puisse se présenter sous toute forme galénique et qui soit stable aussi bien en ce qui concerne la couleur qu'en ce qui concerne le dégagement de CO₂.

La demanderesse a maintenant trouvé un système à base de dérivé phosphonique et de métabisulfite permettant de stabiliser l'acide ascorbique. On observe un effet de synergie important du système selon l'invention.

Certes, il est connu d'utiliser des dérivés d'acide phosphonique dans des compositions contenant de la vitamine C (voir EP-A-670157 cité ci-dessus). Il est connu aussi de stabiliser la vitamine C par du métabisulfite (voir JP-A-6-247855). Toutefois, la synergie de l'association revendiquée n'avait jamais été observée.

La présente invention a donc pour objet un système pour stabiliser l'acide ascorbique, caractérisé par le fait qu'il comprend au moins un dérivé d'acide phosphonique et au moins un métabisulfite, le dérivé d'acide phosphonique et le métabisulfite étant en une quantité suffisante pour agir en synergie.

La présente invention a aussi pour objet l'utilisation du système défini ci-dessus, pour stabiliser l'acide ascorbique.

Les dérivés d'acide phosphonique utilisables dans l'invention peuvent être notamment choisis parmi l'acide éthylènediamine tétra(méthylène phosphonique), l'acide hexaméthylènediamine tétra(méthylène phosphonique), l'acide diéthylènetriamine penta(méthylènephosphonique), et leurs sels et notamment leurs sels de sodium comme le sel pentasodique de l'acide éthylènediamine tétra(méthylène phosphonique).

De manière avantageuse, on utilise l'acide éthylènediamine tétra(méthylène phosphonique), notamment celui vendu par la Société MONSANTO sous la dénomination Dequest 2041. On peut aussi utiliser avantageusement le sel pentasodique de ce dernier, vendu sous la dénomination Dequest 2046 par la Société MONSANTO.

Le dérivé d'acide phosphonique doit être dans une composition le contenant, en une quantité suffisante pour assurer le résultat escompté. Il peut être présent en une quantité allant, par exemple, de 0,005 à 5 % et, de préférence, de 0,05 à 1 % du poids total de la composition.

Le métabisulfite peut être un sel alcalin, alcalino-terreux ou d'ammonium de l'acide anhydrosulfureux. On utilise de préférence le métabisulfite de sodium ou de potassium. Le métabisulfite doit être présent dans une composition le contenant, en une quantité suffisante pour atteindre le résultat escompté. Cette quantité peut aller, par exemple, de 0,005 à 5 % et, de préférence, de 0,05 à 1 % du poids total de la composition.

Le rapport en poids entre le métabisulfite et le dérivé d'acide phosphonique peut aller de 1 à 1000 et, de préférence, de 1 à 5.

Le système selon l'invention permet une stabilisation efficace de l'acide ascorbique. La stabilité de l'acide ascorbique est encore améliorée quand la composition le contenant présente une phase aqueuse ayant un pH allant de 5,5 à 7,5 et en particulier, un pH de 6.

On observe alors une bonne stabilité de l'acide ascorbique, aussi bien pour l'évolution de la couleur que pour la génération de dioxyde de carbone.

Aussi, l'invention a encore pour objet une composition contenant de l'acide ascorbique, caractérisée en ce qu'elle contient le système indiqué ci-dessus.

Ainsi qu'indiqué ci-dessus, la composition selon l'invention contient de préférence de 0,005 à 5 % en poids de dérivé d'acide phosphonique par rapport au poids total de la composition, et de 0,005 à 5 % en poids de métabisulfite par rapport au poids total de la composition.

La composition contenant le système selon l'invention constitue plus particulièrement une composition à application topique, notamment cosmétique et/ou dermatologique, et comporte un milieu physiologiquement acceptable, c'est-à-dire compatible avec la peau, le cuir chevelu et/ou les cheveux.

L'acide ascorbique est présent dans la composition selon l'invention en une quantité appropriée selon l'utilisation envisagée. Il est de préférence présent en une concentration allant de 0,01 à 20 % et mieux de 0,5 à 10 % en poids par rapport au poids total de la composition.

La composition de l'invention peut se présenter sous toutes les formes galéniques normalement utilisées pour une application topique, notamment sous forme d'une solution aqueuse, hydroalcoolique ou huileuse, d'une émulsion huile-dans-eau ou eau-dans-huile ou multiple, d'un gel aqueux ou huileux, d'un produit anhydre liquide, pâteux ou solide, d'un produit à deux phases, d'une dispersion d'huile dans une phase aqueuse à l'aide de sphérules, ces sphérules pouvant être des nanoparticules polymériques telles que les nanosphères et les nanocapsules ou mieux des vésicules lipidiques de type ionique et/ou non-ionique.

Cette composition peut être plus ou moins fluide et avoir l'aspect d'une crème blanche ou colorée, d'une pommade, d'un lait, d'une lotion, d'un sérum, d'une pâte, d'une mousse. Elle peut éventuellement être appliquée sur la peau sous forme d'aérosol. Elle peut également se présenter sous forme solide, et par exemple sous forme de stick.

La composition selon l'invention peut comprendre tous les adjuvants classiquement utilisés dans le domaine cosmétique ou dermatologique, dans des concentrations habituelles. Ces adjuvants sont en particulier choisis parmi les corps gras, les conservateurs, les gélifiants, les parfums, les émulsionnants et tensioactifs, l'eau, les antioxydants, les charges, les actifs (hydrophiles ou lipophiles), les filtres, les solvants, les matières colorantes et leurs mélanges.

Comme corps gras utilisables dans l'invention, on peut citer les huiles minérales (huile de vaseline), les huiles d'origine végétale (huile d'abricot), les huiles d'origine animale, les huiles de synthèse, les huiles de silicone (cyclométhicone, phényltriméthicone) et les huiles fluorées. On peut aussi utiliser des alcools gras, des acides gras, des cires, des résines et leurs mélanges.

Comme émulsionnants utilisables dans l'invention, on peut citer, par exemple, les émulsionnants siliconés tels que les diméthiconecopolyols et les alkyldiméthiconecopolyols. Comme diméthiconecopolyol, on peut citer le mélange de diméthiconecopolyol et de cyclométhicone vendu sous la dénomination "Q2-3225C" par la Société DOW CORNING et le produit vendu sous la dénomination "SF-1228" par la Société GENERAL ELECTRIC. Comme alkyldiméthiconecopolyol, on peut citer le lauryldiméthiconecopolyol tel que, par exemple, celui vendu sous la dénomination "Q2-5200" par la Société DOW CORNING, et le cétyldiméthiconecopolyol tel que, par exemple, celui vendu sous la dénomination "ABIL EM 90" par la Société GOLDSCHMIDT.

Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles, et comme gélifiants lipophiles, on peut citer les argiles modifiées comme les bentones, les sels métalliques d'acides gras, la silice hydrophobe et les polyéthylènes.

Comme actifs, on peut utiliser notamment les hydratants tels que les polyols (glycérine, propylène glycol), les vitamines, les agents kératolytiques et/ou desquamants (acide salicylique et ses dérivés, alpha-hydroxyacides), les agents anti-inflammatoires, les agents apaisants et leurs mélanges. En cas d'incompatibilité, ces actifs peuvent être incorporés dans des sphérules, notamment des vésicules ioniques ou non-ioniques et/ou des nanoparticules (nanocapsules et/ou nanosphères) de manière à les isoler les uns des autres dans la composition.

Comme charge, la composition selon l'invention peut contenir, par exemple, du nylon, de l'amidon et ses dérivés.

En outre, pour améliorer encore la stabilité de la composition contenant l'acide ascorbique, on peut ajuster le pH de la phase aqueuse de la composition à une valeur allant de 5,5 à 7,5 et, en particulier à pH 6. Pour ajuster le pH, on peut ajouter à la composition tout agent basique approprié, choisi notamment parmi les bases minérales comme les hydroxydes de métaux alcalins (hydroxydes de sodium et de potassium) ou les hydroxydes d'ammonium, et les bases organiques, notamment les bases amphotères, c'est-à-dire des bases ayant à la fois des groupements fonctionnels anioniques et cationiques.

Les bases amphotères peuvent être des amines organiques primaires, secondaires, tertiaires ou cycliques et plus spécialement des acides aminés. A titre d'exemple de bases amphotères, on peut citer la glycine, la lysine, l'arginine, la taurine, l'histidine, l'alanine, la valine, la cystéïne, la trihydroxyméthylaminométhane (TRISTA), la triéthanolamine.

L'agent basique est utilisé en une quantité suffisante pour amener le pH de la composition entre 5,5 et 7,5 et de préférence à pH 6. Cette quantité peut aller par exemple de 0,1 à 5 % et de préférence de 0,5 à 2 % du poids total de l'émulsion.

On peut ajouter à l'agent basique une faible quantité d'un acide permettant d'obtenir un effet tampon, tel que l'acide citrique. Cet acide peut être utilisé par exemple en une quantité allant de 0,1 à 5 % du poids total de la composition.

La composition selon l'invention peut constituer notamment une composition de nettoyage, de protection, de traitement ou de soin de la peau du visage et/ou du corps, des muqueuses et/ou des fibres kératiniques, c'est-à-dire des cheveux et/ou des cils.

Aussi, l'invention a encore pour objet l'utilisation cosmétique de la composition selon l'invention pour nettoyer et/ou traiter et/ou protéger la peau et/ou les muqueuses et/ou les fibres kératiniques.

L'invention a aussi pour objet l'utilisation de la composition selon l'invention, pour la préparation d'une crème ou pommade destinée au traitement dermatologique de la peau humaine.

L'invention a encore pour objet un procédé cosmétique et/ou dermatologique de la peau, des muqueuses et/ou des fibres kératiniques, caractérisé en ce qu'il consiste à appliquer sur la peau, les muqueuses et/ou les fibres kératiniques, une composition telle que définie ci-dessus.

### Test

Ce test montre la stabilisation de l'acide ascorbique par le système selon l'invention. On prépare une solution aqueuse de pH 6, contenant 2% en poids d'acide ascorbique. A 10 ml de cette solution, on ajoute :
- Composition A (exemple comparatif): 0,05% en poids de Dequest 2046
- Composition B (exemple comparatif) : 0,05% en poids de métabisulfite de sodium
- Composition C (selon l'invention) : 0,05% en poids de Dequest 2046 + 0,05% de métabisulfite de sodium.

On évalue l'évolution de couleur (ΔDO) de ces compositions conservées pendant 3 jours à 45 °C dans les flacons bouchés, par mesure de la densité optique à 405 nm, par rapport à la solution témoin ne contenant ni Dequest 2046 ni métabisulfite. Les résultats sont rassemblés dans le tableau ci-dessous.

| Composition | ΔDO |
|---|---|
| Composition A (exemple comparatif) | - 13,5% |
| Composition B (exemple comparatif) | + 76,06% |
| Composition C (selon l'invention) | - 38,54% |

Ce tableau montre une diminution de l'évolution de la couleur avec la composition A, une augmentation de l'évolution de la couleur avec la composition B et une nette diminution de l'évolution de la couleur avec la composition C selon l'invention.

Par conséquent, on observe un effet synergie du système selon l'invention pour stabiliser l'acide ascorbique.

Les exemples ci-après de compositions selon l'invention, sont donnés à titre d'illustration et sans caractère limitatif. Les quantités y sont données en % en poids, sauf mention contraire.

### Exemple 1: Composition pour l'éclat du teint

*Phase A :*
   - Mélange de diméthiconecopolyol et de cyclométhicone (Q2-3225C de Dow Corning) 20 %
   - Phényltriméthicone (Dow Corning 556 Fluid) 4 %
   - Huile d'abricot 3 %
*Phase B :*
   - Nylon-12 5 %
*Phase C:*
   - Glycérine 23 %
   - Vitamine C 5 %
   - Métabisulfite de sodium 0,05 %
   - Dequest 2041 0,05 %
   - Galactose 0,33 %
   - Glucose 0,33 %
   - Mannose 0,33 %
   - Propylène glycol 6 %
   - Hydroxyde de sodium 1,93 %
   - Acide citrique 1,24 %
   - conservateur 0,2 %
   - Eau déminéralisée qsp 100 %

Mode opératoire : On prépare la phase aqueuse à température ambiante ; on prépare également la phase huileuse à température ambiante et on y ajoute le nylon. Puis, on verse lentement la phase aqueuse dans la phase huileuse sous agitation rapide.

On obtient une émulsion eau-dans-huile blanche, apte à améliorer l'éclat du teint de la peau et à lisser les traits du visage.

## Revendications

1. Système pour stabiliser l'acide ascorbique, caractérisé en ce qu'il comporte au moins un dérivé d'acide phosphonique et au moins un métabisulfite, le dérivé d'acide phosphonique et le métabisulfite étant en une quantité suffisante pour agir en synergie.

2. Système selon la revendication 1, caractérisé en ce que le rapport en poids entre le métabisulfite et le dérivé d'acide phosphonique va de 1 à 1000.

3. Système selon la revendication 1 ou 2, caractérisé en ce que le dérivé d'acide phosphonique est choisi parmi l'acide éthylènediamine tétra(méthylène phosphonique), l'acide hexaméthylènediamine tétra(méthylène phosphonique), l'acide diéthylènetriamine penta(méthylènephosphonique), leurs sels et leurs mélanges.

4. Système selon l'une quelconque des revendications précédentes, caractérisé en ce que le dérivé d'acide phosphonique est l'acide éthylènediamine tétra(méthylène phosphonique).

5. Système selon l'une quelconque des revendications précédentes, caractérisé en ce que le dérivé d'acide phosphonique est le sel de sodium de l'acide éthylènediamine tétra(méthylène phosphonique).

6. Système selon l'une quelconque des revendications précédentes, caractérisé en ce que le métabisulfite est choisi parmi les sels alcalins, alcalino-terreux, d'ammonium de l'acide anhydrosulfureux, et leurs mélanges.

7. Système selon l'une quelconque des revendications précédentes, caractérisé en ce que le métabisulfite est le métabisulfite de sodium.

8. Composition contenant de l'acide ascorbique, caractérisée en ce qu'elle contient le système selon l'une quelconque des revendications 1 à 7.

9. Composition selon la revendication 8, caractérisée en ce qu'elle constitue une composition à application topique.

10. Composition selon la revendication 8 ou 9, caractérisée en ce qu'elle contient de 0,005 à 5% en poids de dérivé d'acide phosphonique par rapport au poids total de la composition.

11. Composition selon l'une des revendications 8 à 10, caractérisée en ce qu'elle contient de 0,005 à 5% en poids de métabisulfite par rapport au poids total de la composition.

12. Composition selon l'une quelconque des revendications 8 à 11, caractérisée en ce qu'elle contient de 0,01 à 20 % en poids d'acide ascorbique par rapport au poids total de la composition.

13. Composition selon l'une quelconque des revendications 8 à 12, caractérisée en ce qu'elle comporte une phase aqueuse ayant un pH allant de 5,5 à 7,5.

14. Composition selon l'une quelconque des revendications 8 à 12, caractérisée en ce qu'elle comporte une phase aqueuse de pH 6.

15. Composition selon la revendication 13 ou 14, caractérisée en ce que le pH de la phase aqueuse est ajustée à l'aide d'un agent basique choisi parmi les bases minérales et les bases organiques.

16. Composition selon l'une quelconque des revendications 8 à 15, caractérisée en ce qu'elle comprend, en outre, au moins un adjuvant choisi parmi l'eau, les corps gras, les conservateurs, les gélifiants, les tensioactifs et émulsionnants, les antioxydants, les charges, les solvants, les parfums, les matières colorantes, les filtres, les actifs et leurs mélanges.

17. Utilisation du système selon l'une quelconque des revendications 1 à 7 pour stabiliser l'acide ascorbique.

18. Utilisation cosmétique de la composition selon l'une quelconque des revendications 8 à 16, pour nettoyer et/ou traiter et/ou protéger la peau et/ou les muqueuses et/ou les fibres kératiniques.

19. Utilisation de la composition selon l'une quelconque des revendications 8 à 16, pour la préparation d'une crème ou pommade destinée au traitement dermatologique de la peau humaine.

20. Procédé de traitement cosmétique de la peau, des muqueuses et/ou des fibres kératiniques, caractérisé en ce qu'il consiste à appliquer sur la peau, les muqueuses et/ou les fibres kératiniques, une composition selon l'une quelconque des revendications 8 à 16.

## Claims

1. System for stabilizing ascorbic acid, characterized in that it contains at least one phosphonic acid derivative and at least one metabisulphite, the phosphonic acid derivative and the metabisulphite being in a sufficient amount to act in synergy.

2. System according to Claim 1, characterized in that the weight ratio between the metabisulphite and the phosphonic acid derivative ranges from 1 to 1000.

3. System according to Claim 1 or 2, characterized in that the phosphonic acid derivative is chosen from ethylenediaminetetra (methylenephosphonic acid), hexamethylenediaminetetra(methylenephosphonic acid) and diethylenetriaminepenta (methylenephosphonic acid), their salts and their mixtures.

4. System according to any one of the preceding claims, characterized in that the phosphonic acid derivative is ethylenediaminetetra(methylenephosphonic acid).

5. System according to any one of the preceding claims, characterized in that the phosphonic acid derivative is the sodium salt of ethylenediaminetetra(methylenephosphonic acid).

6. System according to any one of the preceding claims, characterized in that the metabisulphite is chosen from the alkali-metal, alkaline-earth metal and ammonium salts of anhydrosulphurous acid, and mixtures thereof.

7. System according to any one of the preceding claims, characterized in that the metabisulphite is sodium metabisulphite.

8. Composition containing ascorbic acid, characterized in that it contains the system according to any one of Claims 1 to 7,

9. Composition according to Claim 8, characterized in that it constitutes a composition for topical application.

10. Composition according to Claim 8 or 9, characterized in that it contains from 0.005 to 5% by weight of phosphonic acid derivative relative to the total weight of the composition.

11. Composition according to one of Claims 8 to 10, characterized in that it contains from 0.005 to 5% by weight of metabisulphite relative to the total weight of the composition.

12. Composition according to any one of Claims 8 to 11, characterized in that it contains from 0.01 to 20% by weight of ascorbic acid relative to the total weight of the composition.

13. Composition according to any one of Claims 8 to 12, characterised in that it contains an aqueous phase with a pH ranging from 5.5 to 7.5.

14. Composition according to any one of Claims 8 to 12, characterized in that it contains an aqueous phase of pH 6.

15. Composition according to Claim 13 or 14, characterized in that the pH of the aqueous phase is adjusted using a basic agent chosen from inorganic bases and organic bases.

16. Composition according to any one of Claims 8 to 15, characterized in that it also comprises at least one adjuvant chosen from water, fatty substances, preserving agents, gelling agents, surfactants and emulsifiers, antioxidants, fillers, solvents, fragrances, dyestuffs, screening agents and active agents, and mixtures thereof.

17. Use of the system according to any one of Claims 1 to 7, to stabilize ascorbic acid.

18. Cosmetic use of the composition according to any one of Claims 8 to 16, for cleansing and/or treating and/or protecting the skin and/or mucous membranes and/or keratin fibres.

19. Use of the composition according to any one of Claims 8 to 16, for the preparation of a cream or ointment intended for the dermatological treatment of human skin.

20. Cosmetic process for treating the skin, mucous membranes and/or keratin fibres, characterized in that it consists in applying a composition according to any one of Claims 8 to 16 to the skin, the mucous membranes and/or the keratin fibres.

## Patentansprüche

1. System zur Stabilisierung von Ascorbinsäure, dadurch gekennzeichnet, daß es mindestens ein Phosphonsäurederivat und mindestens ein Metabisulfit enthält, wobei das Phosphonsäurederivat und das Metabisulfit in einem Mengenanteil vorliegen, der ausreichend ist, um synergistisch zu wirken.

2. System nach Anspruch 1, dadurch gekennzeichnet, daß das Gewichtsverhältnis von Metabisulfit und Phosphonsäurederivat im Bereich von 1 bis 1000 liegt.

3. System nach Anspruch 1 oder 2, dadurch gekennzeichnet, daß das Phosphonsäurederivat unter Ethylendiamintetramethylenphosphonsäure, Hexamethylendiamintetramethylenphosphonsäure, Diethylentriaminpentamethylenphosphonsäure, ihren Salzen und . ihren Gemischen ausgewählt ist.

4. System nach einem der vorhergehenden Ansprüche, dadurch gekennzeichnet, daß es sich bei dem Phosphonsäurederivat um Ethylendiamintetramethylenphosphonsäure handelt.

5. System nach einem der vorhergehenden Ansprüche, dadurch gekennzeichnet, daß es sich bei dem Phosphonsäurederivat um das Natriumsalz von Ethylendiamintetramethylenphosphonsäure handelt.

6. System nach einem der vorhergehenden Ansprüche, dadurch gekennzeichnet, daß das Metabisulfit unter den Alkalisalzen, Erdalkalisalzen und Ammoniumsalzen von Anhydroschwefligsäure sowie deren Gemischen ausgewählt ist.

7. System nach einem der vorhergehenden Ansprüche, dadurch gekennzeichnet, daß es sich bei dem Metabisulfit um Natriummetabisulfit handelt.

8. Zusammensetzung, die Ascorbinsäure enthält, dadurch gekennzeichnet, daß sie ein System nach einem der Ansprüche 1 bis 7 enthält.

9. Zusammensetzung nach Anspruch 8, dadurch gekennzeichnet, daß es sich um eine Zusammensetzung zur topischen Anwendung handelt.

10. Zusammensetzung nach Anspruch 8 oder 9, dadurch gekennzeichnet, daß sie 0,005 bis 5 Gew.-% Phosphonsäurederivat, bezogen auf das Gesamtgewicht der Zusammensetzung, enthält.

11. Zusammensetzung nach einem der Ansprüche 8 bis 10, dadurch gekennzeichnet, daß sie 0,005 bis 5 Gew.-% Metabisulfit, bezogen auf das Gesamtgewicht der Zusammensetzung, enthält.

12. Zusammensetzung nach einem der Ansprüche 8 bis 11, dadurch gekennzeichnet, daß sie 0,01 bis 20 Gew.-% Ascorbinsäure, bezogen auf das Gegamtgewicht der Zusammensetzung, enthält.

13. Zusammensetzung nach einem der Ansprüche 8 bis 12, dadurch gekennzeichnet, daß sie eine wäßrige Phase mit einem pH-Wert im'Bereich von 5,5 bis 7,5 aufweist.

14. Zusammensetzung nach einem der Ansprüche 8 bis 12, dadurch gekennzeichnet, daß sie eine wäßrige Phase mit einem pH-Wert von 6 aufweist.

15. Zusammensetzung nach Anspruch 13 oder 14, dadurch gekennzeichnet, daß der pH-Wert der wäßrigen Phase mit einem Mittel zum Alkalischmachen eingestellt wird, däs unter den anorganischen und organischen Basen ausgewählt ist.

16. Zusammensetzung nach einem der Ansprüche 8 bis 15, dadurch gekennzeichnet, daß sie ferner mindestes einen Zusatzstoff enthält, der unter Wasser, Fettsubstanzen, Konservierungsmitteln, Gelbildnern, grenzflächenaktiven Stoffen und Emulgatoren, Antioxidantien, Füllstoffen, Lösemitteln, Parfums, Färbemitteln, Filtern, Wirkstoffen und deren Gemischen ausgewählt ist.

17. Verwendung des Systems nach einem der Ansprüche 1 bis 7 zur Stabilisierung von Ascorbinsäure.

18. Kosmetische Verwendung der Zusammensetzung nach einem der Ansprüche 8 bis 16 zur Reinigung und/oder zur Behandlung und/oder zum Schutz der Haut und/oder der Schleimhäute und/oder der Keratinfasern.

19. Verwendung der Zusammensetzung nach einem der Ansprüche 8 bis 16 zur Herstellung einer Creme oder Pomade, die zur dermatologischen Behandlung der menschlichen Haut bestimmt ist.

20. Verfahren zur kosmetischen Behandlung der Haut, der Schleimhäute und/oder der Keratinfasern, dadurch gekennzeichnet, daß es darin besteht, eine Zusammensetzung nach einem der Ansprüche 8 bis 16 auf die Haut, die Schleimhäute und/oder die Keratinfasern aufzutragen.
